(19) Europäisches Patentamt
European Patent Office
Office européen des brevets

(11) Numéro de publication: **0 115 985 B1**

(12) **FASCICULE DE BREVET EUROPEEN**

(45) Date de publication du fascicule du brevet: **18.12.85**

(51) Int. Cl.[4]: **C 07 C 17/26**, C 07 C 25/02

(21) Numéro de dépôt: **84400159.4**

(22) Date de dépôt: **25.01.84**

(54) **Procédé d'alkylation de dérives benzéniques halogènes et/ou trifluorométhyles.**

(30) Priorité: **02.02.83 FR 8301594**

(43) Date de publication de la demande: **15.08.84 Bulletin 84/33**

(45) Mention de la délivrance du brevet: **18.12.85 Bulletin 85/51**

(84) Etats contractants désignés: **AT BE CH DE FR GB IT LI LU NL SE**

(56) Documents cités:
**GB - A - 794 153**
**US - A - 3 297 771**

**Le dossier contient des informations techniques présentées postérieurement au dépôt de la demande et ne figurant pas dans le présent fascicule.**

(73) Titulaire: **RHONE-POULENC SPECIALITES CHIMIQUES, "Les Miroirs" 18, Avenue d'Alsace, F-92400 Courbevoie (FR)**

(72) Inventeur: **Soula, Gérard, 33, rue Nungesser, F-69330 Meyzieu (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

**EP 0 115 985 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a pour objet un procédé d'alkylation de composés benzéniques halogénés et/ou trifluorométhylés. Elle concerne plus particulièrement la réaction d'un tel composé benzénique avec un halogénure d'alkyle.

Les composés benzéniques concernés par la présente invention sont les dihalogénobenzènes et les trihalogénobenzènes, l'un au moins des atomes d'halogène pouvant être remplacé par un groupement trifluorométhyle ayant au moins un atome d'hydrogène dont les deux positions ortho sont occupées par des atomes d'halogène ou un groupement trifluorométhyle. Le composé benzénique peut comporter en outre un substituant ne réagissant pas dans les conditions de la réaction.

L'art antérieur décrit la réaction d'alkylation de composés benzéniques par réaction de Friedel et Crafts en catalyse acide (MARCH. Advanced Organic Chemistry 1977). La réaction a lieu préférentiellement en position para: c'est ainsi que lorsqu'on alkyle le métadichlorobenzène, on obtient majoritairement le dichloro-2,4 alkylbenzène et minoritairement le dichloro-2,6 alkylbenzène. D'autre part, en catalyse acide, on assiste à un réarrangement de la chaîne alkyle de l'agent alkylant lorsque cette dernière est suffisamment longue pour que ce réarrangement soit possible. C'est ainsi que lorsque l'agent alkylant est par exemple le bromure de n-butyle, le radical alkyle qui se fixe sur le noyau benzénique est le radical isobutyle.

On constate donc que les procédés de l'art antérieur présentent, dans certains cas, le double inconvénient, d'une part, d'interdire l'accès majoritaire à un composé correspondant à une sélectivité en ortho et, d'autre part, de perturber l'agencement de la chaîne alkyle qui se fixe sur le noyau benzénique.

C'est ce double inconvénient que permet de pallier le procédé mis au point par la titulaire.

La présente invention a pour objet un procédé d'alkylation de dérivés benzéniques halogénés et/ou trifluorométhylés, caractérisé en ce que l'on fait réagir un dérivé benzénique comportant deux ou trois substituants choisis parmi le groupe comprenant les radicaux halogéno et le radical trifluorométhyle et possédant un atome d'hydrogène dont les deux positions ortho sont occupées par deux desdits substituants avec un halogénure d'alkyle en présence d'au moins un amidure alcalin et d'au moins un agent complexant le cation de l'amidure alcalin, le complexe formé étant soluble dans le milieu réactionnel et permettant la réaction avec l'halogénure d'alkyle.

Le procédé de l'invention se démarque très nettement de l'art antérieur dans la mesure où il substitue à une catalyse en milieu acide une catalyse en milieu basique.

On préfère utiliser un amidure choisi parmi l'amidure de sodium, l'amidure de potassium et l'amidure de lithium. L'amidure de sodium est plus particulièrement préféré.

Selon un premier mode de mise en œuvre particulier de l'invention, le composé complexant le cation de l'amidure alcalin est un polyéther macrocyclique ayant de 15 à 30 atomes dans le cycle et constitué de 5 à 10 unités -O-X dans lesquelles X est soit $-CHR_1-CHR_2-$ soit $-CHR_1-CHR_4-CR_3R_2-$, $R_1$, $R_2$, $R_3$, et $R_4$ identiques ou différents étant un atome d'hydrogène ou un radical alkyle ayant de 1 à 4 atomes de carbone, un des X pouvant être $-CHR_1-CHR_4-CR_3R_2-$ quand les unités -O-X comprennent le groupement $-O-CHR_1-CHR_2$.

Selon un deuxième mode de mise en œuvre particulier de l'invention, le composé complexant le cation de l'amidure alcalin est un composé macrocyclique ou bicyclique de formule générale Ia ou Ib.

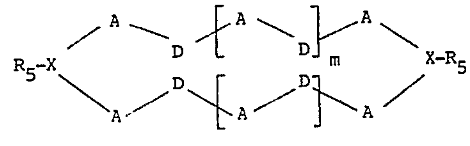
(Ia)

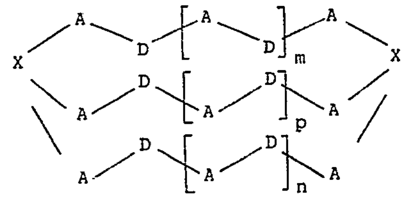
(Ib)

dans lesquelles:

. X représente N ou P

. A représente un groupement alkylène ayant de 1 à 3 atomes de carbone

. D représente O, S ou $N-N_6$ où $R_6$ représente un radical alkyle ayant de 1 à 6 atomes de carbone;

. $R_5$ représente un radical alkyle ayant de 1 à 6 atomes de carbone, n, m et p identiques ou différents sont des nombres entiers compris entre 0 et 5.

Selon un troisième mode de mise en œuvre particulier de l'invention, le composé complexant le cation de l'amidure alcalin est une amine de formule générale:

$$N[CHR_7CHR_8-O-(CHR_9-CHR_{10}-O)_s-R_{11}]_3 \quad (II)$$

dans laquelle s est un nombre entier de préférence supérieur ou égal à 0 et inférieur ou égal à 10 ($0 \leqslant s \leqslant 10$), $R_7$, $R_8$, $R_9$ et $R_{10}$ identiques ou différents représentent un atome d'hydrogène ou un radical alkyle ayant de 1 à 4 atomes de carbone et $R_{11}$ représente un radical alkyle ou cycloalkyle ayant de 1 à 12 atomes de carbone, un radical phényle ou un radical $-C_qH_{2q}-O$ ou $C_qH_{2q+1}-O-$, où q est compris entre 1 et 12.

Selon un quatrième mode de mise en œuvre particulier de l'invention, le composé complexant le cation de l'amidure alcalin est un polyéther linéaire de formule générale:

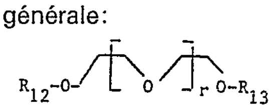
(III)

dans laquelle r est de préférence compris entre 1 et 10 et où $R_{12}$ et $R_{13}$ identiques ou différents représentent un radical alkyle ayant de 1 à 12 atomes de carbone.

Les polyéthers macrocycliques qui peuvent être

mis en œuvre dans le procédé selon l'invention sont connus sous l'appellation générale «d'éthers couronnes» et sont décrits dans le brevet français 6.943.879 publié sous le numéro 2.026.481.

On peut citer comme exemples d'éthers couronnes pouvant être utilisés selon l'invention:

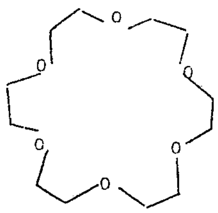
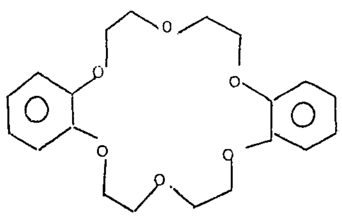
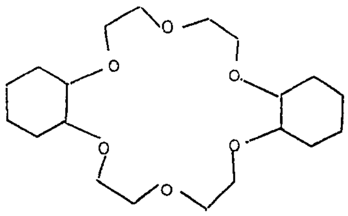
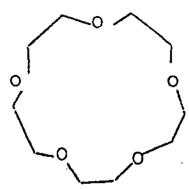

Les composés macrocycliques et bicycliques de formules générales Ia et Ib sont décrits dans le brevet français 7.021.079 publié sous le N° 2.052.947. On peut citer comme exemples de tels composés convenant pour la mise en œuvre du procédé selon l'invention:

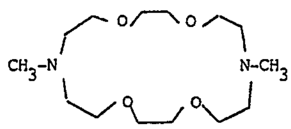
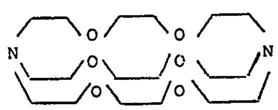
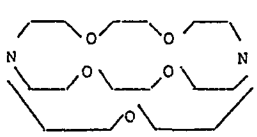
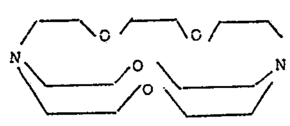
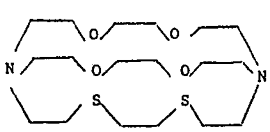
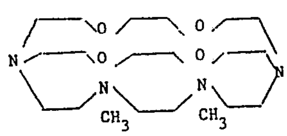
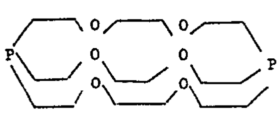

Les amines de formule générale II sont décrites dans la demande de brevet français 7.905.438 publiée sous le N° 2.450.120.

Les amines de formule II préférées sont celles pour lesquelles $R_7$, $R_8$, $R_9$ et $R_{10}$ représentent un atome d'hydrogène ou un radical méthyle, $R_{11}$ et s ayant la signification précédente.

On préfère encore plus particulièrement celles pour lesquelles s est supérieur ou égal à 0 et inférieur ou égal à 6 et $R_{11}$ représente un radical alkyle ayant de 1 à 4 atomes de carbone.

On peut citer comme exemples d'amines de formule II pouvant être utilisées dans le procédé de l'invention les composés suivants:

la tris(oxa-3 heptyl)amine de formule:
$N\text{-}(CH_2\text{-}CH_2\text{-}O\text{-}C_4H_9)_3$

la tris(dioxa-3,6, heptyl)amine de formule:
$N\text{-}(CH_2\text{-}CH_2\text{-}O\text{-}CH_2\text{-}CH_2\text{-}O\text{-}CH_3)_3$

la tris(trioxa-3,6,9 décyl)amine de formule:
$N\text{-}(CH_2\text{-}CH_2\text{-}O\text{-}CH_2\text{-}CH_2\text{-}O\text{-}CH_2\text{-}CH_2\text{-}O\text{-}CH_3)_3$

la tris(dioxa 3,6 octyl)amine de formule:
$N\text{-}(CH_2\text{-}CH_2\text{-}O\text{-}CH_2\text{-}CH_2\text{-}O\text{-}C_2H_5)_3$

la tris(trioxa-3,6,9 undécyl)amine de formule:
$N\text{-}(CH_2\text{-}CH_2\text{-}O\text{-}CH_2\text{-}CH_2\text{-}O\text{-}CH_2\text{-}CH_2\text{-}O\text{-}C_2H_5)_3$

la tris(dioxa-3,6 nonyl)amine de formule:
$N\text{-}(CH_2\text{-}CH_2\text{-}O\text{-}CH_2\text{-}CH_2\text{-}O\text{-}C_3H_7)_3$

la tris(trioxa-3,6,9 dodécyl)amine de formule:
$N\text{-}(CH_2\text{-}CH_2\text{-}O\text{-}CH_2\text{-}CH_2\text{-}O\text{-}CH_2\text{-}CH_2\text{-}O\text{-}C_3H_7)_3$

la tris(dioxa-3,6 décyl)amine de formule:
$N\text{-}(CH_2\text{-}CH_2\text{-}O\text{-}CH_2\text{-}CH_2\text{-}O\text{-}C_4H_9)_3$

la tris(trioxa-3,6,9 tridécyl)amine de formule:
$N\text{-}(CH_2\text{-}CH_2\text{-}O\text{-}CH_2\text{-}CH_2\text{-}O\text{-}CH_2\text{-}CH_2\text{-}O\text{-}C_4H_9)_3$

On peut encore citer:

la tris(dioxa-3,6 méthyl-4 heptyl)amine de formule:
$N\text{-}(CH_2\text{-}CH_2\text{-}O\text{-}\underset{|}{CH}(CH_3)\text{-}CH_2\text{-}O\text{-}CH_3)_3$

la tris(dioxa-3,6 diméthyl-2,4 heptyl)amine de formule:
$N\text{-}(CH_2\text{-}\underset{|}{CH}(CH_3)\text{-}O\text{-}\underset{|}{CH}(CH_3)\text{-}CH_2\text{-}O\text{-}CH_3)_3$

Les polyéthers de formule III préférés sont ceux pour lesquels n est compris entre 1 et 4 et $R_{12}$ représente un radical alkyle ayant de 1 à 6 atomes de carbone.

On peut citer comme exemples de composés de formule III pouvant être mis en œuvre dans le cadre de la présente invention:

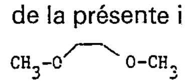
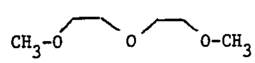 ;
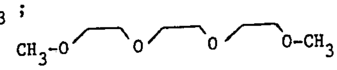
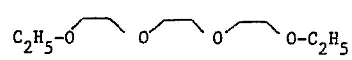 ;
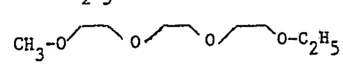
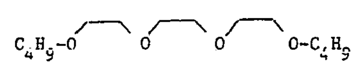
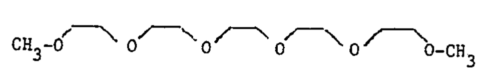

On peut utiliser les divers composés ci-dessus seuls ou en mélange.

Les composés benzéniques de préférence mis en œuvre dans le procédé de l'invention sont représentés par la formule générale:

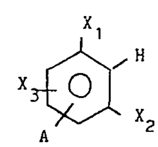 (IV)

dans laquelle $X_1$ et $X_2$ identiques ou différents représentent un atome d'halogène ou un groupement trifluorométhyle, $X_3$ représente un atome d'hydrogène, un atome d'halogène ou un groupement trifluorométhyle et A représente deux ou trois éléments choisis parmi le groupe comprenant l'hydrogène et les radicaux alkyle ayant de 1 à 6 atomes de carbone.

On peut citer comme exemples de composés de formule IV le métadichlorobenzène, le métadifluorobenzène, le métafluorochlorobenzène, le métafluorobromobenzène, le métachlorobromobenzène, le métadibromobenzène, le trichloro-1,3,5 benzène, le trichloro-1,2,4 benzène, le dichloro-3,5 fluorobenzène, le dichloro-2,4 toluène, le métachlorotrifluorométhylbenzène, le métabistrifluorométhylbenzène.

L'halogénure d'alkyle mis en œuvre dans le procédé de l'invention peut être représenté par la formule générale:

$$R(X_4)_n \qquad (V)$$

dans laquelle $X_4$ représente un atome d'halogène, R représente un radical alkyle ayant de 1 à 12 atomes de carbone et n est égal à 1 ou 2.

L'invention concerne donc aussi bien une monoalkylation qu'une dialkylation.

On peut citer comme exemples de composés (V): les halogénures de méthyle, d'éthyle, de propyle, de butyle et les homologues supérieurs, le chlorure et le bromure d'allyle, le chlorure de méthallyle.

On met en œuvre l'invention en utilisant de préférence un rapport molaire de l'amidure alcalin au polyhalogénobenzène au moins égal à 1. Encore plus préférentiellement ce rapport est compris entre 1 et 3.

Le rapport molaire de l'agent complexant le cation de l'amidure alcalin au polyhalogénobenzène est de préférence compris entre 0,01 et 0,2. Encore plus préférentiellement il est compris entre 0,03 et 0,1.

On utilise de préférence un léger excès stœchiométrique d'halogénure d'alkyle. Le rapport molaire de l'halogénure d'alkyle au polyhalogénobenzène sera donc de préférence légèrement supérieur à 1 dans le cas d'une monoalkylation et légèrement supérieur à 2 dans le cas d'une dialkylation.

On peut effectuer la réaction en présence ou en l'absence de solvant.

Le solvant, quand on en utilise un, doit être inerte dans les conditions de la réaction. On peut par exemple utiliser le toluène, le tétrahydrofuranne, le dioxanne, le benzène ou l'éther éthylique.

La température est de préférence comprise entre −40° C et 100° C. Encore plus préférentiellement on opère entre −10 et 60° C.

On opère généralement sous pression atmosphérique, bien que les pressions supérieures ou inférieures à la pression atmosphérique ne soient pas exclues du domaine de l'invention.

Le procédé selon l'invention est basé sur le fait que l'amidure alcalin arrache l'hydrogène situé entre les deux halogènes du composé IV générant ainsi le composé suivant:

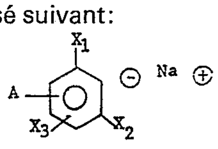

qui est solubilisé dans le milieu réactionnel grâce à la complexation du cation par l'agent complexant, ce qui lui permet de réagir avec l'halogénure d'alkyle pour donner le produit de la réaction:

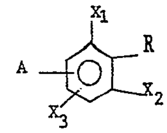

(VI)

Il y a lieu de noter l'intérêt que peut présenter le procédé de l'invention dans certains cas particuliers. Un cas typique est celui du mélange résiduaire de la fabrication de l'orthodichlorobenzène. A côté de ce produit on obtient un mélange de métadichlorobenzène et de paradichlorobenzène qu'il est très difficile de séparer par les méthodes classiques de distillation ou de cristallisation fractionnées.

Jusqu'à présent le métadichlorobenzène contenu dans ce mélange était perdu. L'invention permet de le valoriser par réaction avec un halogénure de méthyle. On obtiendra le dichloro-2,6 toluène produit important à partir du métadichlorobenzène, le paradichlorobenzène ne réagissant pas dans les conditions de l'invention.

L'invention va être maintenant plus complètement décrite à l'aide des exemples qui vont suivre et qui illustrent l'invention sans en limiter la portée.

*Exemple 1*

*Alkylation du trichloro-1,3,5 benzène par le n-bromobutane.*

Dans un réacteur de 200 ml équipé d'un agitateur magnétique, d'une ampoule de coulée et d'un réfrigérant ascendant suivi d'un tube plongeant dans une solution aqueuse d'acide chlorhydrique, on introduit sous azote 7,8 g d'une suspension à 50% d'amidure de sodium dans le toluène. Le mélange est refroidi à 5° C puis on ajoute en 15 min une solution contenant 9,1 g de trichloro-1,3,5 benzène (0,05 mol), 1,6 g de tris(dioxa-3,6 heptyl)amine (0,005 mol) et 40 g de toluène.

Le mélange est maintenu à 17° C sous agitation. On introduit alors en 1 h 13,7 g de bromo-1 butane (0,1 mol). Après ajout de 100 ml d'eau et séparation de la phase organique, celle-ci est analysée par chromatographie en phase vapeur. On observe alors la disparition quasi totale du produit de départ (98%) et la formation d'un produit monoalkylé (73%) et dialkylé (27%).

*Exemple 2*

*Alkylation du métadichlorobenzène par le bromoéthane.*

Avec l'appareillage décrit dans l'exemple précédent, on réalise la réaction suivante:

On introduit sous azote 6 g d'une suspension d'amidure de sodium à 50% dans le toluène. Le mélange est refroidi à 5° C puis on ajoute 7,35 g de métadichlorobenzène (0,05 mol), 0,8 g de tris-(dioxa-3,6 heptyl)amine (0,0025 mol) en solution dans 20 g de toluène. On maintient la température du milieu réactionnel à 18° C et on ajoute en 1 h 8,17 g de bromoéthane (0,075 mol). La réaction se

poursuit pendant 5 h puis on ajoute 100 ml d'eau afin d'arrêter la réaction et de dissoudre tous les sels minéraux présents dans le milieu. La phase organique est récupérée puis séchée. Par chromatographie en phase vapeur on détermine le taux de transformation et la sélectivité en produit monoalkylé (éthyl-1 dichloro-2,6 benzène).

| | |
|---|---|
| Taux de transformation | 80% |
| Sélectivité | 94% |

*Exemple 3*

*Alkylation du métafluorochlorobenzène par le chlorure de méthyle.*

Avec l'appareillage décrit dans l'exemple 1, on réalise la réaction suivante:

On introduit sous azote 8,3 g d'une suspension d'amidure de sodium à 50% dans le toluène. Après refroidissement à 10° C on ajoute 13 g de métachlorofluorobenzène (0,1 mol) et 1,6 g de tris-(dioxa-3,6 heptyl)amine (0,005 mol) dans 20 g de diméthoxyéthane.

On introduit alors du chlorure de méthyle gazeux dans le milieu réactionnel maintenu à 18° C sous agitation.

Après 5 h de réaction, on ajoute 100 ml d'eau afin de détruire l'amidure résiduel et de dissoudre les sels minéraux présents dans le mélange.

La phase organique est décantée, séparée puis séchée en présence de Silicagel. Le toluène est éliminé sous pression réduite et le mélange brut est distillé. On récupère ainsi: 7,3 g de métachlorofluorobenzène non transformé puis 6 g de chloro-2 fluoro-6 toluène (0 = 145-150° C sous 760 mmHg)

*Exemple 4*

*Alkylation du métachlorotrifluorométhylbenzène par le chlorure de méthyle.*

Avec l'appareillage décrit dans l'exemple 3, on réalise la réaction suivante:

On introduit sous azote 5,85 g d'une suspension d'amidure de sodium à 50% dans le toluène. Après refroidissement à 16° C on ajoute 9,07 g de métatrifluorométhylchlorobenzène (0,05 mol) et 0,8 g de tris(dioxa-3,6 heptyl)amine (0,0025 mol) dans 15 g de diméthoxyéthane.

On introduit alors le chlorure de méthyle gazeux dans le milieu réactionnel en maintenant la température vers 23° C sous agitation. Après 6 h de réaction on neutralise par 20 ml d'eau afin de détruire l'amidure résiduel et dissoudre les sels minéraux formés dans le mélange.

Par chromatographie en phase vapeur le taux de transformation trouvé est de 37%.

Le trifluorométhyl-6 chloro-2 toluène est obtenu avec une sélectivité de 90%.

*Exemple 5*

*Alkylation du métabistrifluorométhylbenzène par le chlorure de méthyle.*

Avec l'appareillage décrit dans l'exemple 3, on réalise la réaction suivante:

On introduit sous azote 5,85 g d'une suspension d'amidure de sodium à 50% dans le toluène. Après refroidissement à 15° C on ajoute 10,7 g de métabistrifluorométhylbenzène (0,05 mol) et 0,8 g de tri(dioxa-3,6 heptyl)amine (0,0025 mol) dans 15 g de diméthoxyéthane.

On introduit alors le chlorure de méthyle gazeux dans le milieu réactionnel en maintenant la température vers 20° C sous agitation. Après 4 h de réaction on ajoute 20 ml d'eau afin de détruire l'amidure résiduel et dissoudre les sels minéraux formés dans le mélange.

Par chromatographie en phase vapeur le taux de transformation trouvé est de 16%.

Le bistrifluorométhyl-2,6 toluène est obtenu avec une sélectivité de 86%.

*Exemple 6*

*Alkylation du dichloro-2,4 toluène par le chlorure de méthyle.*

Avec l'appareil décrit dans l'exemple 3, on réalise la réaction suivante:

On introduit sous azote 5,85 g d'une suspension d'amidure de sodium à 50% dans le toluène. Après refroidissement à 16° C on ajoute 8,05 g de dichloro-2,4 toluène (0,05 mol) et 0,8 g de tris-(dioxa-3,6 heptyl)amine (0,0025 mol) dans 15 g de diméthoxyéthane. On introduit dans le milieu réactionnel vers 23° C sous agitation. Après 7 h de réaction, on ajoute 20 ml d'eau afin de détruire l'amidure résiduel et dissoudre les sels minéraux formés dans le mélange. Par chromatographie en phase vapeur le taux de transformation trouvé est de 27% avec une sélectivité de 95%.

*Exemple 7*

*Alkylation du métadichlorobenzène par le chlorure de méthyle.*

Dans l'appareillage décrit dans l'exemple 3, on introduit sous argon 5,85 g d'une suspension d'amidure de sodium à 50% dans le toluène. Après refroidissement à 17° C on ajoute en 10 min 7,35 g de métadichlorobenzène (0,05 mol) et 0,94 g (0,0025 mol) de «kriptofix 222» (1.7.10.16 tétraoxa 4.13 diazacyclooctadécane dans 15 g de toluène.

On introduit alors le chlorure de méthyle gazeux dans le milieu réactionnel en maintenant la température à environ 21° C sous agitation magnétique. Après 18 h de réaction on ajoute 20 ml d'eau afin de détruire l'amidure résiduel et de dissoudre les sels minéraux formés dans le mélange.

Par chromatographie en phase vapeur le taux de transformation trouvé est de 60%.

Le dichloro-2,6 toluène est obtenu avec une sélectivité de 70% en mélange avec le produit de dialkylation: le dichloro-2,6 éthylbenzène (30%).

*Exemple 8*

*Alkylation du métadichlorobenzène par le chlorure de méthyle.*

Dans l'appareillage décrit dans l'exemple 3, on introduit sous argon 5,85 g d'une suspension d'amidure de sodium à 50% dans le toluène. Après refroidissement à 17° C on ajoute en 5 min 7,35 g de métadichlorobenzène (0,05 mol) et 0,7 g de

dicyclohexyl 18 crow 6 (masse molaire: 372 g) dans 15 g de tétrahydrofuranne.

On introduit alors le chlorure de méthyle gazeux dans le milieu réactionnel en maintenant la température à 23° C sous agitation magnétique. Après 23 h de réaction on ajoute 20 ml d'eau afin de détruire l'amidure résiduel et dissoudre les sels minéraux formés dans le mélange. Par chromatographie en phase gazeuse, le taux de transformation trouvé est de 40%.

Le dichloro-2,6 toluène est obtenu avec une sélectivité de 96%.

*Exemple 9*

*Alkylation du métadichlorobenzène par le chlorure de méthyle.*

Dans l'appareillage décrit dans l'exemple 3, on introduit sous argon 5,85 g d'une suspension d'amidure de sodium à 50% dans le toluène. On ajoute en 3 min 7,35 g de métadichlorobenzène (0,05 mol) et 1 g (0,00025 mol) de polyéther, polyéthylèneglycol 400 (diméthyl éther du polyéthylèneglycol) dans 15 g de tétrahydrofuranne.

On introduit alors le chlorure de méthyle gazeux dans le milieu réactionnel en maintenant la température à 22° C sous agitation magnétique. Après 23 h de réaction on ajoute 20 ml d'eau afin de détruire l'amidure résiduel et de dissoudre les sels minéraux formés dans le mélange.

Par chromatographie en phase vapeur le taux de transformation trouvé est de 30%.

Le dichloro-2,6 toluène est distillé sous pression réduite (0 = 120-124° C sous 100 mmHg) après avoir récupéré le métadichlorobenzène non transformé. La sélectivité en dichloro-2,6 toluène est de 94%.

## Revendications

1. Procédé d'alkylation de dérivés benzéniques halogénés et/ou trifluorométhylés, caractérisé en ce que l'on fait réagir un dérivé benzénique comportant deux ou trois substituants choisis parmi le groupe comprenant les radicaux halogéno et le radical trifluorométhyle et possédant un atome d'hydrogène dont les deux positions ortho sont occupées par deux desdits substituants avec un halogénure d'alkyle en présence d'au moins un amidure alcalin et d'au moins un agent complexant le cation de l'amidure alcalin, le complexe formé étant soluble dans le milieu réactionnel et permettant la réaction avec l'halogénure d'alkyle.

2. Procédé selon la revendication 1, caractérisé en ce que le composé complexant le cation de l'amidure alcalin est un polyéther macrocyclique ayant de 15 à 30 atomes dans le cycle et constitué de 5 à 10 unités -O-X dans lesquelles X est soit $-CHR_1-CHR_2-$ soit $-CHR_1-CHR_4-CR_3-R_2-$, $R_1$, $R_2$, $R_3$ et $R_4$ identiques ou différents étant un atome d'hydrogène ou un radical alkyle ayant de 1 à 4 atomes de carbone, un des X pouvant être $-CHR_1-CHR_4-CR_3R_2-$ quand les unités -O-X comprennent le groupement $-O-CHR_1-CHR_2-$.

3. Procédé selon la revendication 1, caractérisé en ce que le composé complexant le cation de l'amidure alcalin est un composé macrocyclique ou bicyclique de formule générale Ia ou Ib:

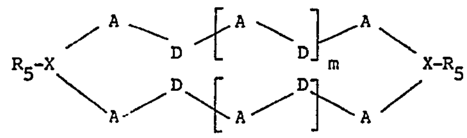
(Ia)

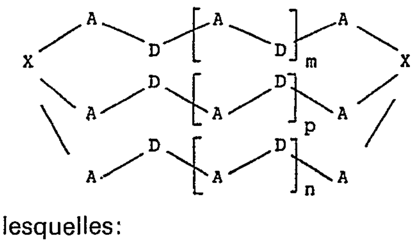
(Ib)

dans lesquelles:

. X représente N ou P

. A représente un groupement alkylène ayant de 1 à 3 atomes de carbone

. D représente O, S ou $N-R_6$ où $R_6$ représente un radical alkyle ayant de 1 à 6 atomes de carbone

. $R_5$ représente un radical alkyle ayant de 1 à 6 atomes de carbone, n, m et p identiques ou différents sont des nombres entiers compris entre 0 et 5.

4. Procédé selon la revendication 1, caractérisé en ce que le composé complexant le cation de l'amidure alcalin est une amine de formule générale:

$$N-[CHR_7-CHR_8-O-(CHR_9-CHR_{10}-O)_s-R_{11}]_3 \quad (II)$$

dans laquelle s est un nombre entier supérieur ou égal à 0 et inférieur ou égal à 10 ($0 \leqslant s \leqslant 10$), $R_7$, $R_8$, $R_9$ et $R_{10}$ identiques ou différents représentent un atome d'hydrogène ou un radical alkyle ayant de 1 à 4 atomes de carbone et $R_{11}$ représente un radical alkyle ou cycloalkyle ayant de 1 à 12 atomes de carbone, un radical phényle ou un radical $-C_qH_{2q}-O$ ou $C_qH_{2q+1}-O-$, où q est compris entre 1 et 12.

5. Procédé selon la revendication 1, caractérisé en ce que le composé complexant le cation de l'amidure alcalin est un polyéther linéaire de formule générale (III)

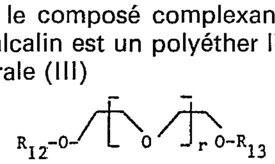
(III)

dans laquelle r est compris entre environ 1 et environ 10 et $R_{12}$ et $R_{13}$ identiques ou différents représentent un radical alkyle ayant de 1 à 12 atomes de carbone.

6. Procédé selon la revendication 2, caractérisé en ce que le polyéther macrocyclique est choisi parmi le groupe comprenant:

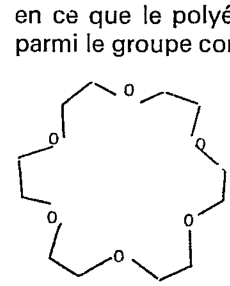
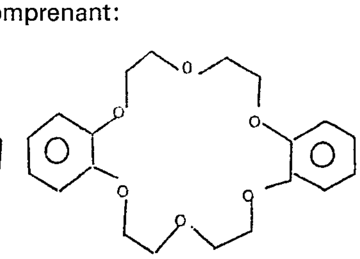

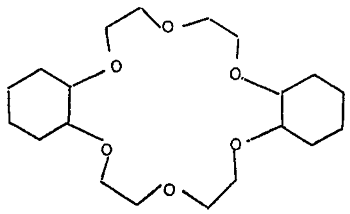

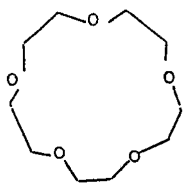

7. Procédé selon la revendication 3, caractérisé en ce que le composé macrocyclique ou bicyclique est choisi parmi le groupe comprenant:

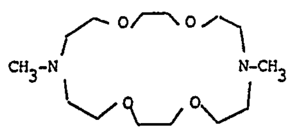

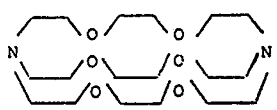

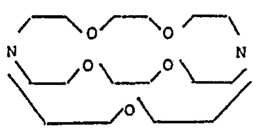

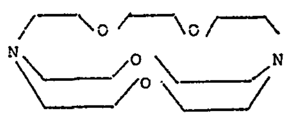

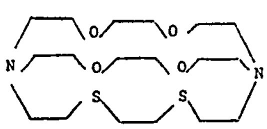

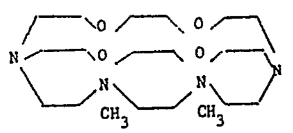

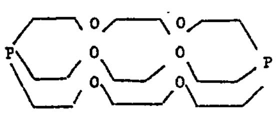

8. Procédé selon la revendication 4, caractérisé en ce que dans la formule III $R_7$, $R_8$, $R_9$ et $R_{10}$ représentent un atome d'hydrogène ou un radical méthyle.

9. Procédé selon la revendication 4, caractérisé en ce que dans la formule II s est supérieur ou égal à 0 et inférieur ou égal à 6 et $R_{11}$ représente un radical alkyle ayant de 1 à 4 atomes de carbone.

10. Procédé selon les revendications 8 et 9, caractérisé en ce que l'amine est la tris(dioxa-3,6 heptyl)amine ou la tris(dioxa-3,6 octyl)amine.

11. Procédé selon la revendication 5, caractérisé en ce que le polyéther linéaire est choisi parmi le groupe comprenant:

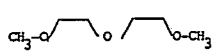

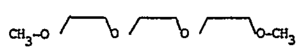

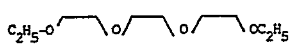

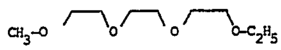

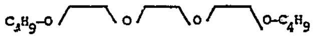

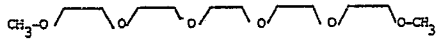

12. Procédé selon l'une quelconque des revendications précédentes, caractérisé en ce que l'amidure alcalin est l'amidure de sodium, de potassium ou de lithium.

13. Procédé selon l'une quelconque des revendications précédentes, caractérisé en ce que le rapport molaire de l'amidure alcalin au polyhalogénobenzène est au moins égal à 1.

14. Procédé selon l'une quelconque des revendications précédentes, caractérisé en ce que le rapport molaire de l'agent complexant le cation de l'amidure alcalin au polyhalogénobenzène est compris entre 0,01 et 0,2.

15. Procédé selon l'une quelconque des revendications précédentes, caractérisé en ce que l'on utilise un léger excès stœchiométrique d'halogénure d'alkyle.

16. Procédé selon l'une quelconque des revendications précédentes, caractérisé en ce que l'on opère à une température comprise entre −40° C et +100° C.

## Patentansprüche

1. Verfahren zur Alkylierung von halogenierten und/oder trifluoromethylierten Benzolderivaten, dadurch gekennzeichnet, dass man ein Benzolderivat, das zwei oder drei Substituenten aus der Gruppe der Halogenoreste und des Trifluoromethylrestes enthält, und ein Wasserstoffatom besitzt, dessen zwei Orthostellungen durch zwei der genannten Substituenten besetzt sind, mit einem Alkylhalogenid in Gegenwart von mindestens einem alkalischen Amid und mindestens einem Komplexbildner für das Kation des alkalischen Amids umsetzt, wobei der gebildete Komplex im Reaktionsmedium löslich ist und die Reaktion mit dem Alkylhalogenid ermöglicht.

2. Verfahren nach Anspruch 1, dadurch gekennzeichnet, dass der Komplexbildner für das Kation des alkalischen Amids ein macrozyclischer Polyäther mit 15 bis 30 Atome im Ring ist, der aus 5 bis 10 O-X-Einheiten besteht, in denen X entweder $-CHR_1-CHR_2-$ oder $-CHR_1-CHR_4-CR_3R_2-$ ist, wobei $R_1$, $R_2$, $R_3$ und $R_4$ identisch oder verschieden, ein Wasserstoffatom oder einen Alkylrest mit 1 bis 4 Kohlenstoffatomen sind und einer der X $-CHR_1-CHR_4-CR_3R_2-$ sein kann, wenn die O-X-Einheiten die Gruppierung $-O-CHR_1-CHR_2$ enthalten.

3. Verfahren nach Anspruch 1, dadurch gekennzeichnet, dass der Komplexbildner für das Kation des alkalischen Amids eine macrozyclische oder eine bizyclische Verbindung der allgemeinen Formel Ia oder Ib ist:

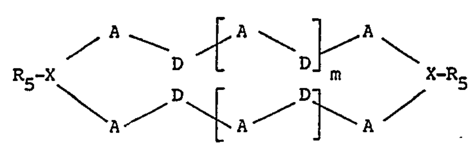 (Ia)

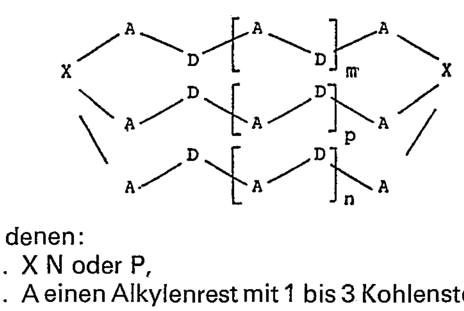 (Ib)

in denen:

. X N oder P,

. A einen Alkylenrest mit 1 bis 3 Kohlenstoffatomen,

. D O, S oder $N-R_6$ darstellt, bei dem $R_6$ einen Alkylrest mit 1 bis 6 Kohlenstoffatomen bedeutet,

. $R_5$ einen Alkylrest mit 1 bis 6 Kohlenstoffatomen darstellt, und n, m und p, identisch oder verschieden ganze Zahlen zwischen 0 und 5 sind.

4. Verfahren nach Anspruch 1, dadurch gekennzeichnet, dass der Komplexbildner für das Kation des alkalischen Amids ein Amin der allgemeinen Formel

$$N\text{-}[CHR_7\text{-}CHR_8\text{-}O\text{-}(CHR_9\text{-}CHR_{10}\text{-}O)_s\text{-}R_{11}]_3 \quad (II)$$

ist, in der s eine ganze Zahl grösser oder gleich 0 und kleiner oder gleich 10 ($0 \leqslant s \leqslant 10$) ist, $R_7$, $R_8$, $R_9$ und $R_{10}$, identisch oder verschieden ein Wasserstoffatom oder einen Alkylrest mit 1 bis 4 Kohlenstoffatomen bedeuten, und $R_{11}$ ein Alkyl- oder Cycloalkylrest mit 1 bis 12 Kohlenstoffatomen, ein Phenylrest oder ein $-C_qH_{2q}-O$ oder $C_qH_{2q+1}-O$-Rest ist, bei dem q zwischen 1 und 12 liegt.

5. Verfahren nach Anspruch 1, dadurch gekennzeichnet, dass der Komplexbildner für das Kation des alkalischen Amids ein linearer Polyäther der allgemeinen Formel:

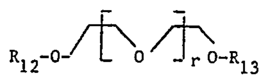 (III)

ist, in der r zwischen etwa 1 und etwa 10 liegt, und $R_{12}$ und $R_{13}$, identisch oder verschieden, einen Alkylrest mit 1 bis 12 Kohlenstoffatomen bedeuten.

6. Verfahren nach Anspruch 2, dadurch gekennzeichnet, dass der macrozyclische Polyäther aus der folgenden Gruppe gewählt ist:

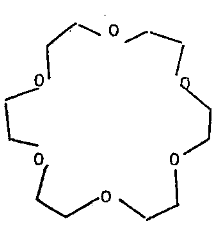

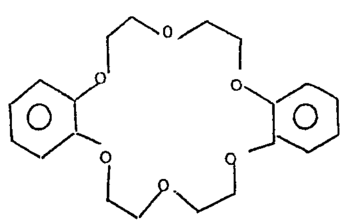

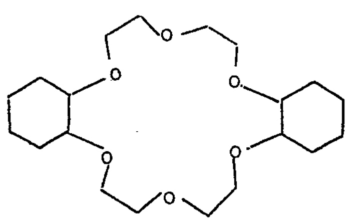

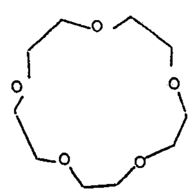

7. Verfahren nach Anspruch 3, dadurch gekennzeichnet, dass die macrozyclische oder bizyclische Verbindung aus der folgenden Gruppe gewählt ist:

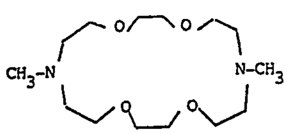

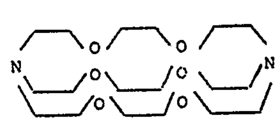

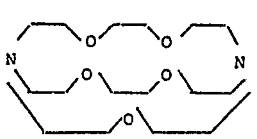

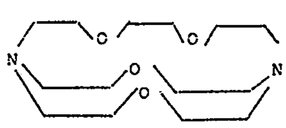

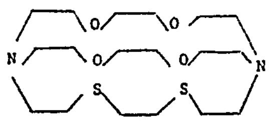

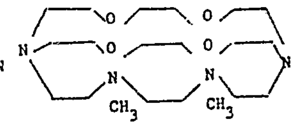

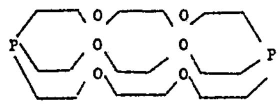

8. Verfahren nach Anspruch 4, dadurch gekennzeichnet, dass $R_7$, $R_8$, $R_9$ und $R_{10}$ in der Formel III ein Wasserstoffatom oder einen Methylrest darstellen.

9. Verfahren nach Anspruch 4, dadurch gekennzeichnet, dass s in der Formel II grösser oder gleich 0 und kleiner oder gleich 6 ist, und $R_{11}$ einen Alkylrest mit 1 bis 4 Kohlenstoffatomen bedeutet.

10. Verfahren nach den Ansprüchen 8 und 9, dadurch gekennzeichnet, dass das Amin das Tris(dioxa-3,6-heptyl)amin oder das Tris(dioxa-3,6 octyl)amin ist.

11. Verfahren nach Anspruch 5, dadurch gekennzeichnet, dass der lineare Polyäther aus der folgenden Gruppe gewählt ist:

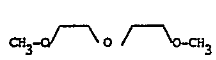

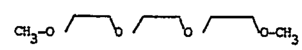

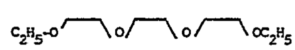

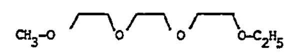

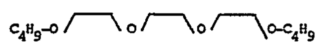

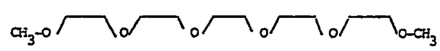

12. Verfahren nach einem der vorhergehenden Ansprüche, dadurch gekennzeichnet, dass das alkalische Amid Natrium-, Kalium- oder Lithiumamid ist.

13. Verfahren nach einem der vorhergehenden Ansprüche, dadurch gekennzeichnet, dass das Molverhältnis des alkalischen Amids zum Polyhalogenbenzol mindestens gleich 1 ist.

14. Verfahren nach einem der vorhergehenden Ansprüche, dadurch gekennzeichnet, dass das Molverhältnis des Komplexbildners für das Kation des alkalischen Amids zum Polyhalogenbenzol zwischen 0,01 und 0,2 liegt.

15. Verfahren nach einem der vorhergehenden Ansprüche, dadurch gekennzeichnet, dass man einen geringen stöchiometrischen Überschuss an Alkylhalogenid einsetzt.

16. Verfahren nach einem der vorhergehenden Ansprüche, dadurch gekennzeichnet, dass man bei einer Temperatur zwischen −40° C und +100° C arbeitet.

## Claims

1. Process for alkylating halogenated and/or trifluoromethylated benzene derivatives, characterized in that a benzene derivative containing two or three substituents, chosen from the group comprising halogen radicals and the trifluoromethyl radical and possessing a hydrogen atom, in which the two ortho positions are occupied by two of the said substituents, is reacted with an alkyl halide in the presence of at least one alkali metal amide and at least one agent which complexes the cation of the alkali metal amide, the complex formed being soluble in the reaction medium and enabling the reaction with the alkyl halide to take place.

2. Process according to Claim 1, characterized in that the compound which complexes the cation of the alkali metal amide is a macrocyclic polyether having from 15 to 30 atoms in the ring and consisting of 5 to 10 -O-X units in which X is either $-CHR_1-CHR_2-$ or $-CHR_1-CHR_4-CR_3R_2-$, $R_1$, $R_2$, $R_3$ and $R_4$, which may be identical or different, being a hydrogen atom or an alkyl radical having from 1 to 4 carbon atoms, one of the X units being able to be $-CHR_1-CHR_4-CR_3R_2-$ when the -O-X units include the group $-O-CHR_1-CHR_2-$.

3. Process according to Claim 1, characterized in that the compound which complexes the cation of the alkali metal amide is a macrocyclic or bicyclic compound of general formula Ia or Ib:

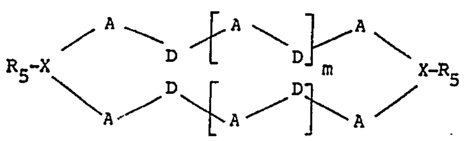
(Ia)

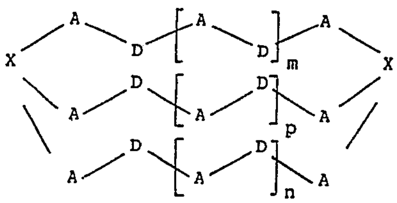
(Ib)

in which:

X denotes N or P

A denotes an alkylene group having from 1 to 3 carbon atoms

D denotes O, S or $N-R_6$, in which $R_6$ denotes an alkyl radical having from 1 to 6 carbon atoms

$R_5$ denotes an alkyl radical having from 1 to 6 carbon atoms, n, m and p, which may be identical or different, being integers between 0 and 5.

4. Process according to Claim 1, characterized in that the compound which complexes the cation of the alkali metal amide is an amine of general formula:

$$N-[CHR_7-CHR_8-O-(CHR_9-CHR_{10}-O)_s-R_{11}]_3 \quad (II)$$

in which s is an integer greater than or equal to 0 and less than or equal to 10 ($0 \leqslant s \leqslant 10$), $R_7$, $R_8$, $R_9$ and $R_{10}$, which may be identical or different, denote a hydrogen atom or an alkyl radical having from 1 to 4 carbon atoms, and $R_{11}$ denotes an alkyl or cycloalkyl radical having from 1 to 12 carbon atoms, a phenyl radical or a $-C_qH_{2q}-O$ or $C_qH_{2q+1}-O-$ radical, in which q is between 1 and 12.

5. Process according to Claim 1, characterized in that the compound which complexes the cation of the alkali metal amide is a linear polyether of general formula (III)

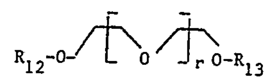
(III)

in which r is between approximately 1 and approximately 10, and $R_{12}$ and $R_{13}$, which may be identical or different, denote an alkyl radical having from 1 to 12 carbon atoms.

6. Process according to Claim 2, characterized in that the macrocyclic polyether is chosen from the group which comprises:

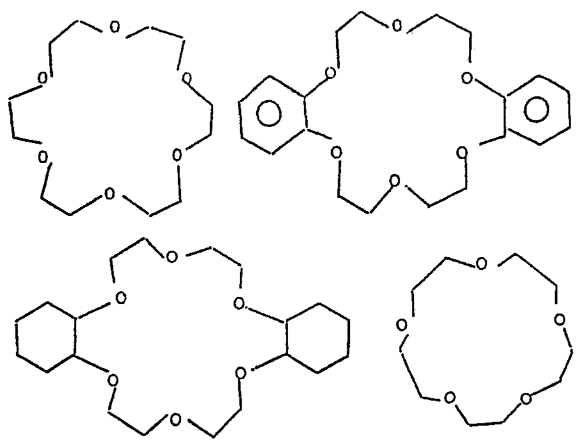

7. Process according to Claim 3, characterized in that the macrocyclic or bicyclic compound is chosen from the group which comprises:

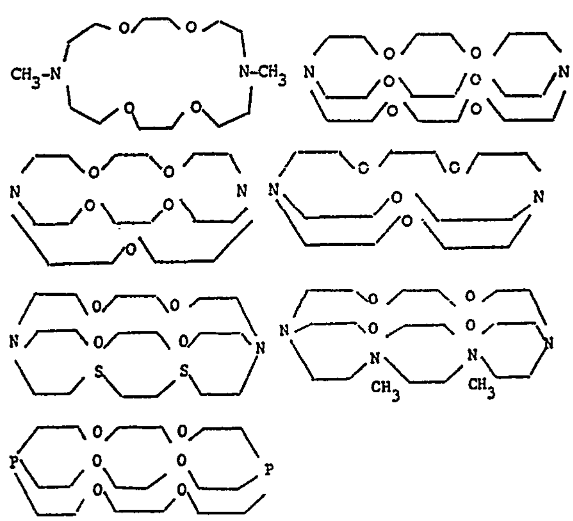

8. Process according to Claim 4, characterized in that, in the formula III, $R_7$, $R_8$, $R_9$ and $R_{10}$ denote a hydrogen atom or a methyl radical.

9. Process according to Claim 4, characterized in that, in the formula II, s is greater than or equal to 0 and less than or equal to 6, $R_{11}$ denotes an alkyl radical having from 1 to 4 carbon atoms.

10. Process according to Claims 8 and 9, characterized in that the amine is tris(3,6-dioxaheptyl)amine or tris(3,6-dioxaoctyl)amine.

11. Process according to Claim 5, characterized in that the linear polyether is chosen from the group which comprises:

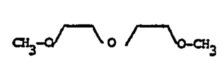

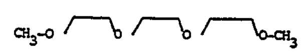

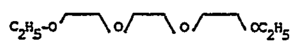
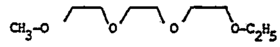

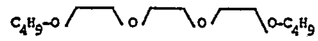

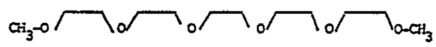

12. Process according to any one of the preceding claims, characterized in that the alkali metal amide is sodium amide, potassium amide or lithium amide.

13. Process according to any one of the preceding claims, characterized in that the mole ratio of the alkali metal amide to the polyhalobenzene is equal to at least 1.

14. Process according to any one of the preceding claims, characterized in that the mole ratio of the agent which complexes the cation of the alkali metal amide to the polyhalobenzene is between 0.01 and 0.2.

15. Process according to any one of the preceding claims, characterized in that a slight stoichiometric excess of alkyl halide is used.

16. Process according to any one of the preceding claims, characterized in that the reaction is performed at a temperature between −40° C and +100° C.